# EUROPÄISCHE PATENTSCHRIFT

(11) **EP 4 081 147 B1**
(45) Veröffentlichungstag und Bekanntmachung des Hinweises auf die Patenterteilung: **08.10.2025**
(21) Anmeldenummer: 20845569.1
(22) Anmeldetag: 22.12.2020
(51) Int. Cl.: A61B 18/14, A61B 18/00

(54) **VORRICHTUNGEN ZUR ENUKLEATION INTRAKORPORALER GEWEBEBEREICHE**
DEVICES FOR THE ENUCLEATION OF INTRACORPOREAL TISSUE REGIONS
DISPOSITIFS D'ÉNUCLÉATION DE ZONES TISSULAIRES INTRACORPORELLES

(30) Priorität: 23.12.2019 DE 102019220537
(43) Veröffentlichungstag der Anmeldung: 02.11.2022
(73) Patentinhaber: Gimmi GmbH, 78532 Tuttlingen (DE)
(72) Erfinder: RUFFIEUX, Kurt, 6003 Luzern (CH); MIERNIK, Arkadiusz, 79110 Freiburg (DE)
(74) Vertreter: Westphal, Mussgnug & Partner Patentanwälte mbB
(86) Internationale Anmeldenummer: PCT/EP2020/087639
(87) Internationale Veröffentlichungsnummer: WO 2021/130229

(56) Entgegenhaltungen:
- WO-A1-03/024349
- DE-T2- 69 119 177
- DE-U1- 29 913 688
- GB-A- 2 308 979

## Beschreibung

### Technisches Gebiet

Die Erfindung bezieht sich auf eine Vorrichtung zur Enukleation intrakorporaler Gewebebereiche, insbesondere der Prostata, mit einer Sonde, an deren distalem Ende wenigstens ein frei zugänglicher Elektrodenkörper angebracht ist, der über wenigstens eine in Längserstreckung der Sonde verlaufende elektrische Leitung mit elektrischer Energie beaufschlagbar ist.

### Stand der Technik

Vorrichtungen zur Enukleation intrakorporaler Gewebebereiche werden vor allem zum Zwecke der Behandlung von gutartigen Vergrößerungen der Prostata, so genannte benigne Prostatahyperplasie (BPH), eingesetzt. Weit verbreitet hierzu sind endoskopische Instrumente, die minimalinvasiv transurethal, d.h. durch die Harnröhre hindurch, intrakorporal an den Ort der zu behandelnden Prostata positioniert werden, um gutartige vergrößerte Prostatabereiche komplett oder inkomplett zu entfernen. Zur Durchführung des auch als transurethrale Prostatatresektion, kurz TURP, bezeichneten minimalinvasiven, chirurgischen Eingriffes dienen so genannte Resektoskope, siehe beispielsweise die Druckschrift DE 935391 B, die zum Zwecke eines lokalen Gewebeabtrages wenigstens einen Arbeitskanal aufweisen, durch den ein sondenartig ausgebildeter Elektrodenkatheter führbar ist, der eine schlingenförmig ausgebildete, distale Elektrodenspitze aufweist, die mit HF-Wechselstrom beaufschlagbar ist, der zu einer Erhitzung der Elektrodenschlinge führt, mit der lokale thermisch induzierte Gewebeschichtabtrennungen möglich sind. Um eine gute optische Kontrolle während der Gewebeablation zu gewährleisten, wird während der Operation kontinuierlich Spülflüssigkeit über das Resektoskop eingebracht und wieder abgesaugt. Die Spülflüssigkeit ist bei Verwendung so genannter monopolarer Resektionen elektrolytfrei, wohingegen bei Verwendung bipolarer Resektoskope isotonische Kochsalzlösungen als Spülflüssigkeit zum Einsatz kommen. Die HF-mono- oder bipolare Resektion der Prostata wird als Goldstandard bei der Behandlung von BPH angesehen, gleichwohl weist sie auch einige Nachteile auf. So ist mit dieser Behandlungsmethode die Resektion des sich an der Prostata gutartig ausbildenden Drüsengewebes zumeist nur unvollständig und die zur Gewebeentnahme erforderlichen Eingriffszeiten sind insbesondere bei größeren Adenomen lang und führen für den Patienten zu einer erheblichen Belastung. Überdies führt eine im Rahmen einer monopolaren Resektoskopie erforderliche Spülung des im Wege der TURP behandelten Gewebebereiches mit hypotoner Spüllösung fallweise zu einem so genannten TUR-Syndrom, das zu Einschwemmungen in das Gefäßsystem führt und mögliche Komplikationen, wie Lungenödem, Hirnödem, Hämolyse und Nierenversagen nach sich ziehen kann.

Neuere transurethale endoskopische Enukleationstechniken unter Einsatz von laserbasierten Resektoskopen, insbesondere unter Verwendung der so genannten Holmium-Laserenukleation der Prostata, kurz HoLEP, zeigen bessere klinische Ergebnisse. Die laserbasierte Prostataenukleation ermöglicht neben der Behandlung auch sehr großer Adenome insbesondere eine schichtengerechte Präparation, wodurch eine schonende Schichtablösung zwischen der Prostatakapsel und der vergrößerten Innendrüse bzw. dem Adenom möglich wird. Dieses Vorgehen ermöglicht ein besonders blutarmes Operieren im Vergleich zu konventionellen Techniken, die auf dem Einsatz der vorstehend erläuterten HF-Elektrodenschlingen beruhen. Im Unterschied zu den bekannten konventionellen Techniken bietet die laserbasierte Enukleation eine vollständige, feingewebliche Abtrennung und Aufarbeitung des zu entfernenden Gewebes.

Aufgrund der hohen Kosten und der großvolumig bauenden Geräte, die es im Operationssaal entsprechend zu platzieren gilt, ist die Verfügbarkeit und Unterbringung derartiger Systeme nur an wenigen Orten möglich.

Die Druckschrift US 10,470,837 B2 beschreibt eine Vorrichtungen sowie ein Verfahren zur Behandlung von Rhinitis, d.h. chronische Nasenschleimhautentzündung, mit der es möglich ist Nervenäste von sich in der hinteren Nasenhöhle befindlichen Nasennerven zu ablatieren. Eine flexibel ausgestaltete, chirurgische Sonde ermöglicht ihre Einführung und Positionierung in den submukösen Raum einer lateralen Nasenwand, wo die Neuroablation des hinteren Nasennervs mit der chirurgischen Sonde durchgeführt werden kann. Die Sondenvorrichtung verfügt an ihrer distalen Sondenspitze über ein Leuchtmittel, das das submuköse Gewebe durchleuchtet, um es dem Chirurgen zu ermöglichen, die submuköse Position des distalen Endes der chirurgischen Sonde vom Inneren der Nasenhöhle zu visualisieren. In einer Ausführungsform ist die Sondenspitze abgerundet ausgebildet und weist eine HF-Elektrodenanordnung zur lokalen Erzeugung Joulscher Wärme auf.

Die Druckschrift DE 696 36 885 T2 offenbart ein Chirurgiesystem mit gekühlter Elektrodenspitze. Längs des hohlen Elektrodenkörper, dessen distales Ende spitz oder abgerundet ausgebildet sein kann, ist ein Kühlsystem integriert, durch das die vermittels der Elektrodenanordnung am distalen Bereich an die intrakorporale Umgebung applizierbare Wärmemenge kontrollierbar ist, um Überhitzungseffekte zu vermeiden.

Die Druckschrift DE 10 2015 119 694 A1 beschreibt ein Elektrochirurgiesystem zur Resektion von Körpergewebe, das wenigstens zwei Elektroden umfasst, die zu Zwecken einer kurzlebigen Gasblasenbildung innerhalb einer mit dem Elektrochirurgiesystem in Kontakt stehenden Flüssigkeit mittels einer Hochspannungsversorgungseinheit mit elektrischen Hochspannungspulsen beaufschlagt werden.

Die in der Druckschrift DE 88 07 820 U1 erläuterte Elektrodenstimulationssonde besitzt eine Form und Größe, die an die anatomischen Gegebenheiten des menschlichen Schließmuskelbereiches angepasst sind, zur Behandlung von Blasen- oder Analinkontinenz sowie von Hämorrhoiden.

Die Druckschrift WO 02/098312 A2 offenbart eine Sondenanordnung mit einer distalen Sondenspitze, in Art einer Punktionsnadelspitze, zur elektrothermischen Koagulation von Gewebe, die zwei Elektroden vorsieht, von denen eine mit einem Innenleiter und die andere mit einem Aussenleiter verbunden sind. Innen- und Aussenleiter der Sondenanordnung sind elektrisch voneinander isoliert. Der Innenleiter ist ferner derart gewählt, dass die Biegesteifigkeit der Sondenanordnung erhöht wird.

Der Druckschrift DE 10 2017 100 409 A1 ist eine elektrochirurgische Vorrichtung zu entnehmen, die einen Handgriff und einen daran kontrolliert drehbar angebrachten Schaft aufweist.

Aus der Druckschrift GB 2 308 979 A ist ein elektrochirurgisches Instrument zur Behandlung der Prostata bekannt. Ferner ist aus der Druckschrift WO 03/024349 A1 ein weiteres elektrochirurgisches Instrument bekannt.

### Darstellung der Erfindung

Der Erfindung liegt die Aufgabe zugrunde, eine Vorrichtung zur Enukleation intrakorporaler Gewebebereiche, insbesondere der Prostata mit einer Sonde, an deren distalem Ende wenigstens ein frei zugänglicher Elektrodenkörper angebracht ist, der über wenigstens eine in Längserstreckung der Sonde verlaufende elektrische Leitung mit elektrischer Energie beaufschlagbar ist, derart weiterzubilden, so dass die Nachteile, die bisher mit der HF-mono- oder bipolaren Resektion der Prostata verbunden sind, vermieden und vergleichbare operative Eigenschaften und Resultate, wie sie mit der laserbasierten endoskopischen Enukleation der Prostata, kurz EEP, erzielbar sind, möglich werden sollen. Es soll insbesondere eine kostengünstige und qualitativ gleichwertige Alternative zu der apparativ aufwendigen laserbasierten EEP geschaffen werden.

Lösungsgemäß zeichnet sich die Vorrichtung zur Enukleation intrakorporaler Gewebebereiche, insbesondere zur Therapierung benigner Prostatathyperplasie mit den Merkmalen der unabhängigen Ansprüche 1, 2 und 12 aus.

Der lösungsgemäßen Vorrichtung liegt die Idee zugrunde, die Handhabung und therapeutische Wirkung eines Laserkatheters, der zum Zwecke der transurethalen Prostataenukleation ausgebildet ist, durch eine zur elektrischen Ablation und Koagulation von Gewebeschichten bzw. -bereichen befähigte Sonde, die gleichfalls in Verbindung mit einem Resektoskop eingesetzt werden kann, weitgehend vollständig nachzubilden.

Der am distalen Ende der Sonde angebrachte, kuppenförmig ausgebildete Elektrodenkörper ist hierzu derart gestaltet, dass die Elektrodenkörperoberfläche über gleitende Eigenschaften verfügt, d.h. vollumfänglich glatt und ohne Hinterschneidungen oder Kanten ausgebildet ist, so dass durch distalwärtigen Vorschub oder seitlichem Bewegen der Sonde vermittels des kuppenförmig, glatt ausgebildeten Elektrodenkörpers eine möglichst atraumatische und stumpfe Präparation des zu therapierenden Gewebebereiches ermöglicht wird. Der Kuppen- bzw. domförmig geformte Elektrodenkörper vermag durch fein dosierten distalen oder seitlichen Vorschub Gewebeschichten bzw. Gewebelagen durch einen reinen mechanischen Verdrängungsprozess ohne oder ohne nennenswerte Läsionsbildung auf- bzw. abzutrennen.

Der lösungsgemäßen Sondenausbildung liegt unter anderem die Erkenntnis zugrunde, dass der schonende, laserbasierte Gewebeantrennungsprozess, bspw. HoLEP, im Wesentlichen von der Strahlqualität und -impuls des vom distalen Laserkatheterende emittierten Laserstrahls abhängt. Neben der Strahlleistung, dem Strahldurchmesser, der Strahldivergenz sowie Wellenlänge und Frequenz spielen die Strahlintensitätsverteilung und die Strahlwechselwirkung mit dem Energie absorbierenden Medium längs des Strahlquerschnittes eine Rolle bei der Interaktion zwischen Laserstrahl, gegebenenfalls der resultierenden Bildung von Gasblasen und den zu penetrierenden bzw. präparierenden Gewebebereichen zum Zwecke einer präzisen, zeiteffizienten und schonenden Ablösung des Adenoms von der Prostata.

Laserstrahlen hoher Güte weisen eine minimale Strahldivergenz auf und verfügen über eine Strahlintensitätsverteilung längs des Strahlquerschnittes in Form einer Gaus-Kurve, weshalb derartige Laserstrahlen auch als Gausstrahlen bezeichnet werden. Diese räumliche Strahlintensitätsverteilung und das spezifische Muster der räumlichen Energieverteilung am Ort der Energieapplikation erscheinen nach derzeitiger Kenntnis die Voraussetzungen für die mit der laserbasierten Prostataenukleation verbundenen Vorteile zu sein, die die Inspiration und Grundlage für die räumliche Ausbildung der lösungsgemäß ausgebildeten Elektrodenoberfläche des am distalen Endes der Sonde angebrachten Elektrodenkörpers bildet.

Die kuppenförmige Ausbildung des Elektrodenkörpers ist unter anderem durch die Raumform des Strahlungsintensitätsprofils eines Gaussttrahls inspiriert und motiviert und ermöglicht durch schonendes distales oder seitliches Bewegen der Sonde Gewebebereiche bzw. -schichten stumpf zu präparieren. Da der Elektrodenkörper zusätzlich über wenigstens eine elektrische Leitung mit elektrischer Energie versorgbar ist, kann im Bedarfsfall das Gewebe zusätzlich elektrisch unterstützend durchtrennt, ablatiert und/oder koaguliert werden.

Der vorzugsweise aus Metall oder einer Metalllegierung gefertigte Elektrodenkörper ist entweder in Form einer Monopolar-Elektrode mit einer längs der Sonde geführten elektrischen Leitung, oder in Form einer Bipolar-Elektrode mit zwei längs der Sonde geführten, elektrischen Leitungen elektrisch verbunden. Die neuartige Sonde kann in herkömmlichen Resektoskopen eingesetzt und an üblichen, im OP-Saal vorhandenen HF-Geräten angeschlossen werden. Die lösungsgemäße Vorrichtung ermöglicht eine präzise, zeiteffiziente und schonende Behandlung der Prostata durch die Harnröhre hindurch und erhöht gleichsam, wie bei laserbasierten Resektoskopen die operative Sicherheit und vermag signifikant die Komplikationsraten im Vergleich zu konventionellen elektrischen Prostataresektionstechniken (TURP) zu senken. Die lösungsgemäße Vorrichtung ist darüber hinaus kostengünstig und besitzt das Potential positive und gesundheitsökonomische Auswirkungen auf das gesamte Gesundheitssystem zu entfalten.

Vorzugsweise ist die kuppenförmig ausgebildete Elektrodenoberfläche des Elektrodenkörpers zumindest längs des ersten axialen Abschnittes in Form einer räumlichen Strahlungsintensitätsverteilung eines Laserstrahls mit gaußförmiger Intensitätsverteilung, eines Paraboloids oder Ellipsoids nachgebildet. Gleichsam sind auch davon abweichende Raumformen denkbar und realisierbar, bei denen die Querschnittsflächen des Elektrodenkörpers längs des jeweils ersten axialen Abschnittes jeweils von einem Umfangsrand umgeben sind, der entweder ausschließlich gekrümmt geformt ist oder gekrümmte und gerade Umfangsrandabschnitte aufweist, wobei die Übergänge zwischen gekrümmt zu geraden Umfangsrandabschnitten glatt, d.h. stetig differenzierbar ausgebildet sind. Die beschriebene gekrümmte Ausbildung erlaubt dem Operateur durch Drehen des Instrumentes in eine präferierte Richtung zu arbeiten.

Betrachtet man im Gegenzug die orthogonal zu den einzelnen Querschnittsflächen orientierte Längsschnitte durch den Elektrodenkörper, so weist ein bevorzugt ausgebildeter Elektrodenkörper ausschließlich Längsschnitte auf, die jeweils von einer Umfangskontur begrenzt sind, die ebenfalls an jeder Stelle stetig differenzierbar d.h. glatt ausgebildet sind. Vorzugsweise lassen sich die die einzelnen Längsschnitte begrenzenden Umfangskonturen durch einen Teilkreis, eine Parabel, eine Teilellipse oder ein Teiloval beschreiben. Wesentliches Kennzeichen aller lösungsgemäß kuppenförmig ausgebildeter Elektrodenoberfläche ist ihre glatte, nach außen in Erscheinung tretende Formgebung, die dazu geeignet ist, Gewebebereiche, insbesondere Gewebeschichten im Wege eines schonenden, durch die Formgebung des Elektrodenkörpers bestimmten mechanischen Verdrängungsvorganges läsionsfrei oder weitgehend läsionsfrei zu durchtrennen. Scharfkantige Oberflächenkonturen oder Konturen mit geringen Radien, wie sie bei konventionellen Elektrodenschlingen üblich sind, sind ausdrücklich für die Formgestaltung des Elektrodenkörpers ausgeschlossen, um möglichst läsionsfrei zwischen zwei anatomische Gewebeschichten mit Hilfe des Elektrodenkörpers zu gelangen bzw. mechanisch vorzudringen.

In einer weiteren bevorzugten Ausführungsform weist der kuppenförmig ausgebildete Elektrodenkörper im Bereich seines distalen Endes einen mamillenartig ausgeprägten, gleichfalls glatt und kuppenförmig ausgebildeten Fortsatz auf, durch den der mechanische Auftrennprozess beim distalen Vorschub der lösungsgemäß ausgebildeten Sonde in schonender Weise unterstützt wird. Nähere Erläuterungen hierzu können anhand nachfolgender Illustrationen entnommen werden.

An den glatt, kuppenförmig ausgebildeten Elektrodenkörper schließt sich proximalwärts ein zweiter axialer Abschnitt an, längs dessen die orthogonal zur Längserstreckung des Elektrodenkörpers orientierten Querschnittsflächen mit zunehmendem Abstand zum distalen Kuppenende gleichbleiben. Vorzugsweise sind die Querschnittsflächen längs des zweiten axialen Abschnittes kreisförmig ausgebildet, so dass der Elektrodenkörper in diesem Bereich eine geradzylinderförmige Außenform annimmt.

Der lösungsgemäß ausgebildete Elektrodenkörper ist starr mit der Sonde verbunden, die vorzugsweise als Hohlkanüle ausgebildet ist und axiale Schub- bzw. Druckkräfte wie auch Biegekräfte zu übertragen vermag. Die starre Fügung des Elektrodenkörpers an die Hohlkanüle erfolgt vorzugsweise über ein biokompatibles, bioniertes, elektrisch isolierendes Fügemittel in Form eines mechanischen Zwischenstückes beispielsweise aus Keramik, Kunststoff oder Glas oder eines Gußteils, beispielsweise auf Epoxidharzbasis, das sich zu Verbindungszwecken verfestigt hat.

Die als Hohlkanüle ausgebildete Sonde verfügt zudem über eine hohe Biegefestigkeit, um auch bei mechanischen, auf den Elektrodenkörper wirkenden Querbelastungen relativ zur Längserstreckung der Sonde ohne jegliche Formänderungen Stand zu halten. Derartige Querbelastungen können beispielsweise auftreten, wenn der Operateur den Elektrodenkörper seitlich bzw. quer zur Sondenlängserstreckung zum Gewebeabtrag bzw. Gewebeablösung bewegt. In diesem Fall dürfen keine oder nur minimale Deformationen, vorzugsweise längs der gesamten Sonde, aber zumindest in jenem distalen Bereich der Sonde, der zum Zwecke der stumpfen Präparation distalseitig aus dem Resektoskop hinausreicht, auftreten. Um die Formstabilität zu gewährleisten, bietet es sich an, die in Form einer Hohlkanüle ausgebildete Sonde aus einem metallischen Werkstoff, bspw. aus Instrumentenstahl, oder einem robusten biegesteifen, vorzugsweise Faser-verstärkten Kunststoff mit einer geeignet dick gewählten Hohlkanülenwandstärke zu fertigen.

So verfügt die Sonde zumindest im distalen Sondenbereich über eine Steifigkeit bzw. Biegefestigkeit derart, dass bei Einwirken eines Biegemomentes auf die Sonde, das durch eine Krafteinwirkung auf den Elektrodenkörper quer zur Sondenlängserstreckung hervorgerufen wird, während die Sonde in einem Abstand von wenigstens 30 mm von der distalen Sondenspitze mechanisch fest eingespannt ist - das einer typischen Sondenführung längs und durch ein Resektoskop entspricht - die distale Sondenspitze maximal 2 mm quer zur Sondenlängserstreckung ausgelenkt wird und in keinem Fall zu einer plastischen Verformung der Sonde kommt. Hierbei wird bei einer Auslenkung von 2 mm die aufgebrachte Kraft gemessen und das Biegemoment berechnet (F x I). Die Sonde ist dabei derart gestaltet, dass bei einem Biegemoment von wenigstens 0,1 Nm, vorzugsweise von wenigstens 0,2 Nm, besonders vorzugsweise von wenigsten 0,3 Nm, die vorstehenden Anforderungen an die Formstabiliät der Sonde gewährleistet bleibt.

Vorzugsweise sind sämtliche Oberflächen, d.h. sowohl die Oberfläche des Elektrodenkörpers als auch die des Fügemittels sowie der sich daran proximalwärts anschließenden, starren Hohlkanüle reibungsarm, d.h. glatt und oberflächengleitend ausgebildet. Es bietet sich an zumindest die Elektrodenoberfläche des Elektrodenkörpers zu polieren bzw. zu honen.

Die lösungsgemäße Vorrichtung kann bereichsweise oder vollständig mit einer die Gleitfähigkeit erhöhenden Oberflächenvergütung, vorzugsweise in Form einer Beschichtung, versehen sein. Die Beschichtung besteht beispielsweise aus PTFE, Polyurethan, Polysiloxan oder ein Hydrogel, oder einem Polymer mit gleitwiderstandreduzierenden Additiven oder einer Kombination davon .

Durch die mechanisch steife bzw. starre Verbindung zwischen Hohlkanüle, Fügemittel und Elektrodenkörper wird eine mechanische Kraftübertragung längs der Sonde in axialer und seitlicher Richtung auf den Elektrodenkörper möglich, mit dem eine schonende und zielgerichtete Gewebetrennung in Form einer atraumatischen, stumpfen Präparation möglich wird. Unter dem Begriff der "stumpfen Präparation" wird ein Auseinanderdrängen von zwei anatomischen Strukturen verstanden, die miteinander durch eine bindegewebige und/oder vaskularisierte oder avaskularisierte Schicht verbunden sind. Auf diese Weise wird ein artifizieller Raum geschaffen, der unter normalen biophysiologischen und anatomischen Bedingungen ausgebildet werden kann. Der Vorgang der stumpfen Präparation wird ausschließlich durch mechanisches Bewegen in distaler Vorwärtsrichtung, wie auch durch seitliches Hin- und Herschwenken und / oder rotatorische Bewegungen des Elektrodenkörpers um die Längsachse der Sonde, bzw. Hohlkanüle von einem Operateur im intrakorporalen Gewebezielbereich initiiert. Bedarfsweise kann der elektrisch leitende Elektrodenkörper durch Hochfrequenz-Bestromung eine zusätzliche Schneid-Ablations- und/oder Koagulationswirkung innerhalb des Gewebebereiches entfalten.

Um den Vorgang des Auseinanderdrängens von zwei anatomischen Schichtstrukturen möglichst Gewebe-schonend und für den Operateur effektiv und dennoch feindosiert und richtungsselektiv vornehmen zu können, erweisen sich Raumformen für die Ausgestaltung des Elektrodenkörpers längs des ersten Elektrodenabschnittes als besonders geeignet, die von einer Axialsymmetrie um die Längsachse der Sonde abweichen und zweidimensionale Querschnittformen besitzen, die längs einer von zwei orthogonal zueinander orientierten Querschnittsachsen abgeflacht ausgebildet sind, gleichsam der Form eines Spatels. Durch die Abflachung der ansonsten rund, d.h. kantenfrei ausgebildeten Elektrodenkörperform ist ein flächiges Auseinanderdrängen zweier anatomischer Schichtstrukturen besser möglich, indem die jeweils abgeflachten Elektrodenkörperflächen, die jeweils längs einer Querschnittsachse gegenüberliegen, parallel zwischen den zu separierenden Schichtstrukturen beim Sondenvorschub geführt werden.

Vorzugsweise jedoch nicht notwendigerweise sind die abgeflachten Elektrodenkörperflächen symmetrisch zu jeweils einer Querschnittsache ausgebildet, bspw. weisen beide Elektrodenkörperoberflächen wenigstens einen ebenen Flächenbereiche auf oder beide Elektrodenkörperoberflächen weisen wenigstens einen konvex gekrümmten Flächenbereich auf. Ebenso ist es denkbar, dass Elektrodenkörperoberfläche konvex und die andere Elektrodenkörperoberfläche konkav gekrümmt ausgebildet ist oder eine der beiden Elektrodenkörperoberflächen weist wenigstens einen gekrümmten Flächenbereich und die andere Elektrodenkörperoberfläche wenigstens einen ebenen Flächenbereich auf.

Im Falle einer asymmetrischen Abflachung des Elektrodenkörpers weist vorzugsweise eine der beiden Elektrodenkörperflächen in Längserstreckung zur Sondenlängsachse eine geschwungen ausgebildete Flächenform auf, längs der wenigstens ein Krümmungswendepunkt enthalten ist. Die gegenüberliegende Elektrodenkörperfläche hingegen ist vorzugsweise weitgehend geradlinig ausgebildet. Selbstverständlich sind hiervon abweichende jeweils abgeflachte Elektrodenkörperflächenformen denkbar, die ein effektives Vordringen zwischen zwei und Auseinanderdrängen zweier Gewebeschichten ermöglichen.

Durch eine asymmetrische Abflachung des Elektrodenkörpers werden überdies für den Operateur durch wechselweise bidirektionale Rotation des Elektrodenkörpers um die Sondenlängsachse auf das Gewebeumfeld einwirkende, gewebeverdrängende Kippbewegungen beim Vorschub der Sonde möglich, die den Vorgang des Auseinanderdrängens zweier anatomischer Schichtstrukturen fallweise unterstützen können.

Zudem eröffnet sich durch eine asymmetrische Abflachung des Elektrodenkörpers die Möglichkeit der asymmetrischen Ausbildung von mit dem Gewebe in Berührung tretenden Elektrodenkontaktflächen. Bspw. kann die im vorstehenden Ausführungsbeispiel erläuterte weitgehend geradlinig ausgebildete, abgeflachte Elektrodenkörperflächenform elektrisch leitend ausgebildet sein, wohingehend die gegenüberliegend geschwungen ausgebildete Elektrodenkörperflächenform zumindest nicht vollständig elektrisch leitend ausgebildet ist und bspw. lokal mit einer elektrisch isolierenden Schicht überdeckt ist oder umgekehrt.

Die lösungsgemäße Vorrichtung eignet sich insbesondere für einen chirurgischen Einsatz in Verbindung mit einem an sich bekannten Resektoskop, das neben eines Arbeitskanals zur Durchführung der lösungsgemäßen Sonde weitere Arbeits- und/oder Spülkanäle vorsieht, wodurch für den Chirurgen die Durchführung einer optisch überwachbaren transurethralen Prostataenukleation möglich ist. Vorzugsweise ist zum Zwecke einer zentrierten und lagedefinierten Führung der Sonde längs des Arbeitskanals des Resektoskops wenigstens eine Führungshülse längs der Hohlkanüle angebracht, die sowohl als Zentrier- und Gleitelement als auch als Durchführungselement für wenigstens ein weiteres medizinischen Instrument parallel zur Sonde durch den Arbeitskanal dient.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a,b: Seitendarstellung sowie Draufsicht auf eine lösungsgemäß ausgebildete Vorrichtung,
- Fig. 2a,b: Ausführungsbeispiel für einen Elektrodenkörper in Längsschnitt sowie Untersichtdarstellung,
- Fig. 3a,b: Ausführungsbeispiel für einen Elektrodenkörper mit distaler zusätzlicher Kuppenstruktur,
- Fig. 4 - 7: alternative Raumformen zur Ausbildung des Elektrodenkörpers längs des ersten axialen Abschnittes,
- Fig. 8a-e: Längsschnitt a), um 90° gedrehte Längsansicht b) eines distalseitig an der Sonde angebrachten Elektrodenkörpers, sowie Querschnitte c), d), e),
- Fig. 9a-e: Längsschnitt a) und um 90° gedrehte Längsansicht b) einer alternativen Ausgestaltungsform eines distalseitig an der Sonde angebrachten Elektrodenkörpers sowie Querschnitte c), d), e),
- Fig. 10 a-c: Längsschnitt eines distalseitig an der Sonde angebrachten Elektrodenkörpers a), Querschnitte b), c),
- Fig. 11a,b: Längsschnitt eines distalseitig an der Sonde angebrachten Elektrodenkörpers a), Querschnitt b) und
- Fig. 12: Längsschnitt eines distalseitig an der Sonde angebrachten Elektrodenkörpers.

### Wege zur Ausführung der Erfindung, gewerbliche Verwendbarkeit

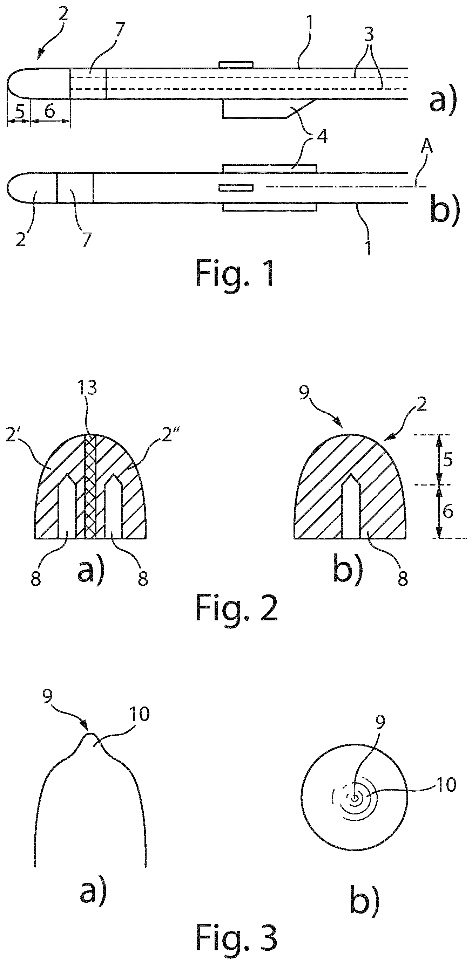

In den Figuren 1a, b ist eine Vorrichtung zur Enukleation intrakorporaler Gewebebereiche in einer Seiten- und Draufsicht dargestellt. Die Vorrichtung weist eine als Hohlkanüle ausgebildeten Sonde 1 auf, an deren distalen Ende ein frei zugänglicher Elektrodenkörper 2 fest angebracht ist. Der Elektrodenkörper 2 ist mit wenigstens einer, vorzugsweise zwei elektrischen Leitungen 3 verbunden, die innerhalb der Sonde 1 proximalwärts längs geführt sind und mit einer nicht dargestellten, elektrischen Energiequelle verbindbar ausgebildet sind. Typsicherweise ist längs der Sonde 1 eine Führungshülse 4 angebracht, die als Zentrier- und Gleitelement innerhalb und längs eines Arbeitskanals eines nicht weiter dargestellten Resektoskops dient und zudem die Durchführung eines medizinischen Instrumentes bspw. eines optischen Leiters ermöglicht. Der vorzugsweise aus Metall oder einem metallischen Werkstoff bestehende, elektrisch leitende Elektrodenkörper 2 weist wenigstens einen ersten axialen Abschnitt 5 auf, der eine kuppenförmig ausgebildete Elektrodenoberfläche 6 besitzt, die im gezeigten Ausführungsbeispiel sphärisch ausgebildet ist. Der Elektrodenkörper 2 weist gemäß Fig. 1 einen zweiten axialen Abschnitt 6 auf, der geradzylinderförmig ausgebildet ist und sich nahtlos glatt an den ersten axialen Abschnitt 5 anschließt.

Der metallische Elektrodenkörper 2 ist über ein biokompatibles, elektrisch isolierenden Fügemittel 7 fest mit der als Hohlkanüle ausgebildeten der Sonde 1 verbunden. Das Fügemittel 7 ist bspw. als Formkörper zur Verbindung zwischen dem Elektrodenkörper und der Hohlkanüle ausgebildet.

Die Krümmung der Elektrodenoberfläche des Elektrodenkörpers 2 im ersten axialen Abschnitt 5 ist konstruktiv vorgegeben und für den Vorgang eines stumpfen Präparierens optimal gewählt. Im Weiteren werden bevorzugte Elektrodenoberflächen-Geometrien für die Ausbildung des Elektrodenkörpers 2 beschrieben.

In Fig. 2a ist ein Längsschnitt durch einen bipolaren Elektrodenkörper 2 illustriert, der zu Zwecken der elektrischen Kontaktierung zwei elektrische Kontakthülsen 8 vorsieht, in die jeweils die Enden der elektrischen Leitungen 3 einmünden und mit dem Elektrodenkörper 2 fest verbunden sind. Zudem ist der Elektrodenkörper 2 durch eine elektrisch isolierende Zwischenschicht 13 in zwei voneinander elektrisch isolierte Elektrodenkörperhälften 2', 2" getrennt. Figur 2b ist hingegen ein monopolarer Elektrodenkörper 2 dargestellt mit nur einer elektrischen Kontakthülse 8.

Die in den Figuren 2a, b dargestellten Elektrodenkörper 2 weisen jeweils eine sphärisch geformte Kuppenform längs des ersten axialen Abschnittes 5 auf, die in eine geradzylinderförmige Aussenform längs des zweiten axialen Abschnittes 6 nahtlos, glatt übergeht.

Die Querschnittsflächen der jeweils sphärisch längs des ersten axialen Abschnittes 5 ausgebildeten Elektrodenkörper 2 entsprechen Kreisflächen mit jeweils kontinuierlich zunehmenden Kreisdurchmesser bis zu einem Kreisdurchmesser, der dem Durchmesser der geradzylinderförmigen Aussenform längs des zweiten axialen Abschnittes 6 entspricht. Die zugehörigen Längsschnitte durch den Elektrodenkörper 2 stellen somit im ersten axialen Abschnitt 5 Halbkreisflächen dar.

Fig. 3a gibt eine Formvariante zur Ausbildung des Elektrodenkörpers 2 wieder, die im Unterschied zur sphärischen Kuppenform gemäß Fig. 2 a, b zusätzlich eine kleinere Kuppenform 10 aufweist. Die mamillenartig ausgebildete Kuppe 10 dient zur Unterstützung des Auseinanderdrängens zweier anatomischer Strukturen, die miteinander durch eine bindegewebige und/oder vaskularisierte oder avaskularisierte Gewebeschicht verbunden sind.

Die in Fig. 3b illustrierte Ansicht zeigt eine Draufsicht auf das distale Ende 9 des Elektrodenkörpers 2 in proximaler Projektion. Aus dieser Darstellung kann die kreisrunde Ausgestaltung der mamillenartigen Kuppenform entnommen werden. Der Übergang zwischen der mamillenartigen Kuppe 10 und der übrigen Kontur des Elektrodenkörpers 2 innerhalb des ersten axialen Abschnittes 5 erfolgt nahtlos und glatt, d.h. die Oberfläche des Elektrodenkörpers 2 ist an jeder Stelle stetig differenzierbar ausgebildet.

Die Form des kuppenförmig ausgebildeten Elektrodenkörpers 2 kann von der sphärisch kuppenförmigen Ausbildung gemäß der Ausführungsformen der Figuren 1 und 2 abweichen.

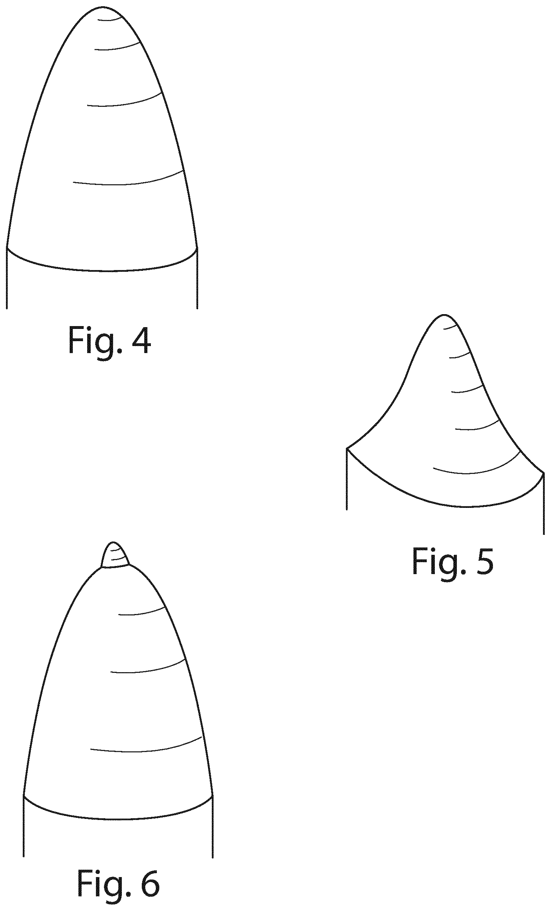

In den Figuren 4 bis 6 sind weitere alternative Raumformen für die Ausgestaltung des Elektrodenkörpers 2 insbesondere längs des ersten axialen Abschnittes 5 gezeigt. Fig. 4 zeigt die Raumform eines Paraboloids, Fig. 5 die Nachbildung einer gausschen Strahlungsintensitätsverteilung, die gleich oder näherungsweise und abschnittsweise die Raumform eines Ellipsoids entspricht.

Fig. 6 gibt die Raumform eines Paraboloids, vergleichbar mit der Darstellung in Fig. 4 wieder, die jedoch ergänzt ist durch eine mamillenartig ausgebildete zusätzliche Kuppenform 10 am distalen Ende des Elektrodenkörpers 2.

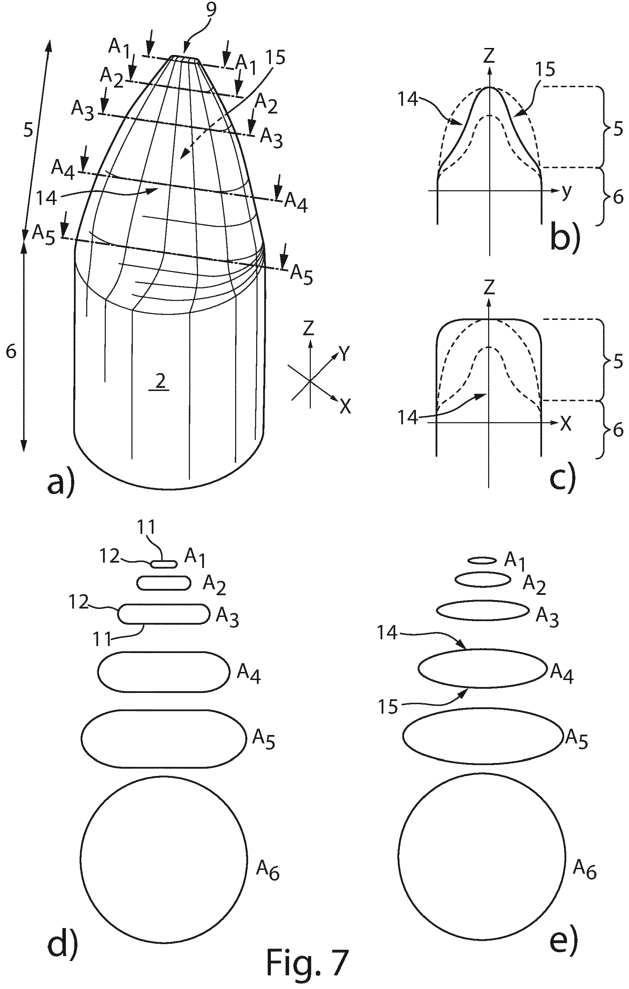

Fig. 7a illustriert in perspektivischer Ansicht eine weitere Ausgestaltungsform für die Formgebung des Elektrodenkörpers 2 zumindest längs des ersten Abschnittes 5. In diesem Fall ist die kuppenförmige Ausbildung des Elektrodenkörpers schaufelartig bzw. rund abgeflacht ausgebildet. Durch die Abflachung des Elektrodenkörpers 2 innerhalb des ersten Abschnittes 5 längs der y-Achse, siehe das in Figur 7a dargestellte x-y-z-Koordinatensystem, bilden sich zwei längs der y-Achse gegenüberliegende, abgeflachte Elektrodenoberflächen 14, 15 aus, die jeweils parallel oder weitgehend parallel zwischen zwei zu separierende Gewebeschichten zum Zwecke des Auseinanderdrängens beider anatomischen Schichtstrukturen geführt werden.

In den Figuren 7b und 7c sind jeweils Schnittbilder durch den Elektrodenkörper 2 jeweils längs der Schnittebene z-y, siehe Figur 7b, und längs der Schnittebene z-x, siehe Figur 7c wiedergegeben, siehe jeweils die durchgezogene Linienführung. Die jeweils strichliert gezeichneten Profilschnitte in den Figuren 7b und 7c stellen in nicht begrenzender Weise Variationen für die Ausbildung der Raumform des in Figur 7a dargestellten Elektrodenkörpers 2 dar.

Zudem zeigen die Figuren 7d und 7e mögliche alternative Querschnittsformen A1 bis A5, in der Abfolge der in Figur 7a angegebenen Schnittebenen.

Im Falle der Querschnittsformen gemäß Fig. 7d weist der Elektrodenkörper 2 im ersten axialen Abschnitt 5 Querschnittsform A1 bis A5 auf, die jeweils einen geradlinigen Abschnitt 11 sowie gekrümmte Abschnitte 12 aufweisen. Mit zunehmenden Abstand zum distalen Ende 9 nähern sich die Querschnittsformen A1 bis A5 etc. einem kreisrunden Querschnitt morphologisch an. Die geradlinigen Abschnitte 11 sind jeweils den abgeflachten Elektrodenoberflächen 14, 15 zugeordnet.

Im Falle der in Fig. 7e illustrierten Querschnittsformen A1 bis A5 handelt es sich um elliptische Querschnitte, deren Querschnittsgrößen sich vom distalen Ende 9 proximalwärts kontinuierlich vergrößern. Die gering gekrümmten Ellipsenabschnitte sind den abgeflachten Elektrodenoberflächen 14, 15 zugeordnet.

Auch in diesem Fall gehen die elliptischen Querschnittsformen morphologisch in einen kreisrunden Querschnitt A6 über, der dem äußeren Hohlkanülenquerschnitt der Sonde 1 entspricht.

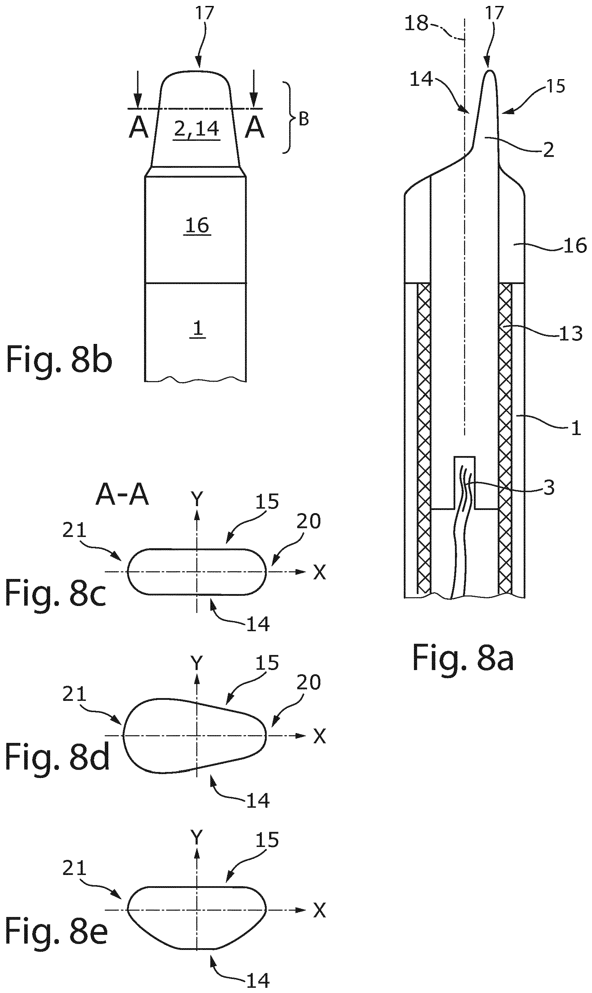

In Figur 8a ist ein Längsschnitt durch eine weitere Ausführungsform zur Enukleation intrakorporaler Gewebebereiche mit einer als starre Hohlkanüle ausgebildeten Sonde 1 dargestellt, an deren distalem Ende ein Elektrodenkörper 2 angebracht ist, der über wenigstens eine in Längserstreckung der Sonde 1 verlaufende elektrische Leitung 3 mit elektrischer Energie beaufschlagbar ist. Der Elektrodenkörper 2 ist über eine innerhalb der Sonde 1 eingebrachte elektrische Isolationsschicht 13 gegenüber der metallischen Sondenwand galvanisch entkoppelt. Zudem umfasst ein elektrisch isolierender Keramik-Hülsenkörper 16 den distalseitig die Hohlkanüle 1 überragenden Elektrodenkörper 2. Der Keramik-Hülsenkörper 16 fügt sich bündig an die Außenkontur der Hohlkanüle 1 an. Der distalseitig über den elektrisch isolierenden Keramik-Hülsenkörper 16 ragende Bereich B des Elektrodenkörpers 2 ist abgeflacht, gleichsam der Form eines Spatels, und weist zwei Elektrodenoberflächen 14, 15 auf, deren Form aus der Zusammenschau der Längsschnittdarstellung gemäß Figur 8a und der gegenüber Figur 8a um 90° gedrehten Seitenlängsdarstellung gemäß Figur 8 b zu entnehmen ist. Mögliche räumliche Ausgestaltungsformen des Elektrodenkörpers 2 sind zudem im Weiteren unter Bezugnahme auf die Figuren 8c bis 8e zu entnehmen.

Das distale Ende 17 des spatelförmig abgeflachten Elektrodenkörpers 2 weist eine distalseitig abgerundete Kontur auf, an die sich nahtlos die zwei Elektrodenoberflächen 14, 15 anschließen. Das distale Ende 17 ist zudem außermittig zur Sondenlängsachse 18 angeordnet. Die Elektrodenoberfläche 15 mündet weitgehend konturerhaltend an der Außenwand des sich proximalwärts ersteckenden Elektrodenkörpers 2. Die Elektrodenoberfläche 14 hingegen ist schaufelförmig gekrümmt ausgebildet und schließt radial nahtlos an den stirnseitigen Rand des Keramik-Hülsenkörpers 16 an, der die Schaufelform konturerhaltend ausgebildet ist.

Im praktischen Einsatz der Sonde sorgen das abgerundete Ende 17 für ein schonendes Verdrängen und Separieren zweier Gewebeschichten. Die Formgebung der beiden Elektrodenoberflächen 14 und 15 sowie die sich daran proximalseitig anschliessenden, stirnseitigen Konturen des Keramik-Hülsenkörpers 16 ermöglichen eine räumliche Beabstandung der getrennten Gewebebereiche.

In Figur 8c ist eine bevorzugte Querschnittsform durch den Elektrodenkörper 2 längs des in Fig. 8b angegebenen Schnittes A-A dargestellt. Beide längs zur y-Querschnittsachse abgeflachten Elektrodenoberflächen 14, 15 sind symmetrisch zur x-Querschnittsachse eben ausgebildet und an ihren längs der y-Achse gegenüberliegenden Flächenenden jeweils durch eine abgerundete, vorzugsweise zylindrisch ausgebildete Oberflächenform 20, 21 verbunden.

Eine alternative Querschnittsform ist in Figur 8 d illustriert. In diesem Fall sind die Elektrodenoberflächen 14, 15 ebenfalls symmetrisch zur x-Querschnittsachse, jedoch eben, aufeinander zulaufend ausgebildet. Die Flächenenden beider Elektrodenoberflächen 14, 15 sind jeweils über unterschiedlich dimensionierte zylindrische Oberflächenformen 20, 21 verbunden, von denen die in der Querschnittsdarstellung gemäß Fig, 8d linke Oberflächenform 21 einen größeren Krümmungsradius besitzt als die gegenüberliegende Oberflächenform 20. Die breitere bzw. dickere, abgerundete Oberflächenform 21 unterstützt bei einer Seitwärtsbewegung der Sonde in Richtung der dickeren Oberflächenform 21 den Trennvorgang zwischen zwei Gewebeschichten unter Vermeidung von Schnittfolgen im Gewebe.

Eine weitere alternative Querschnittsform zeigt Figur 8 e. In diesem Fall ist die Elektrodenoberfläche 15 eben und die gegenüberliegende Elektrodenoberfläche 14 konvex ausgebildet.

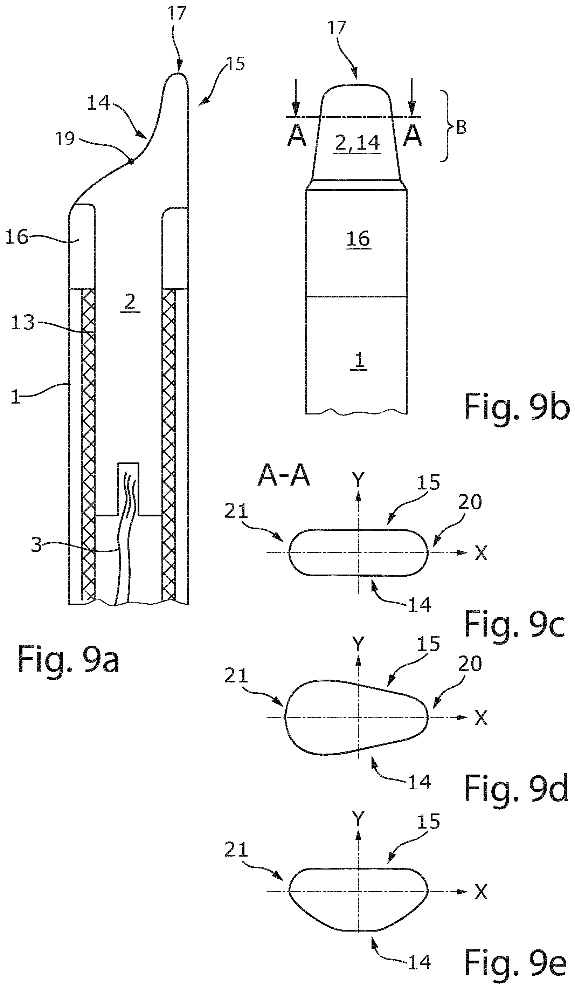

Eine gegenüber der vorstehenden in den Figuren 8a, b gezeigten Ausführungsform modifizierte Ausführungsvariante ist in Figur 9 a, b illustriert, die gleichsam eine Längsschnittdarstellung sowie eine um 90° gedrehte Längsansicht zeigt. In diesem Fall schließt der distalseitig über den Keramik-Hülsenkörper 16 hinausragende Bereich B des Elektrodenkörpers 2 radial und axial bündig an die Außenwand des Keramik-Hülsenkörpers 16 an, wobei die Elektrodenoberfläche 15 axial bündig an die Außenwand des Keramik-Hülsenkörpers 16 angrenzt, wohingegen die Elektrodenoberfläche 14 schaufelförmig geschwungen ausgebildet ist und einen Krümmungswendepunkt 19 aufweist. Auch der in den Figuren 9 a, b gezeigte Elektrodenkörper 2 kann Raumformen annehmen, die durch die in den Figuren 9 c bis 9e dargestellten alternativen Querschnitte vorgegeben sind, gleichsam den in den Figuren 8c bis 8e dargestellten Querschnittsformen.

Der gesamte Oberflächenbereich des Elektrodenkörpers 2 ist bei allen vorstehend illustrierten Ausführungsbeispielen zumindest längs des Bereiches B glatt poliert und/oder gehont. Vorzugsweise sind die Elektrodenoberfläche des Elektrodenkörpers 2, der Keramik-Hülsenkörper 16 sowie die Sonde 1 mit einer reibungsarmen Beschichtung überzogen, vorzugsweise mit einer Beschichtung, die PTFE, TPU, Polysiloxane oder Hydrogel aufweist.

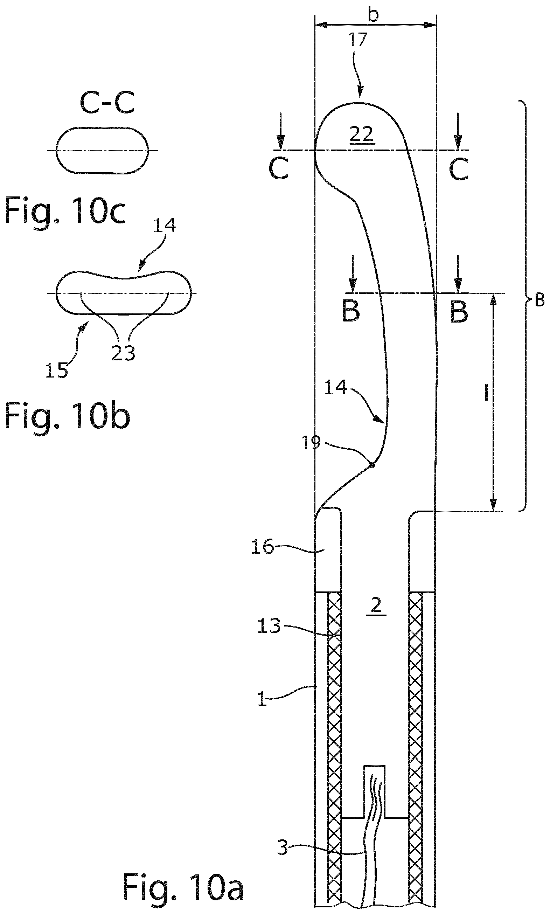

In Figur 10 a ist ein Längsschnitt durch ein weiteres Ausführungsbeispiel einer lösungsgemäßen Vorrichtung dargestellt. Um Wiederholungen zu vermeiden sind jene Komponenten, die baugleich und/oder gleichwirkend mit bereits erläuterten Komponenten ausgebildet sind, mit bereits eingeführten und erläuterten Bezugszeichen versehen.

Der aus einem elektrisch leitenden und formstabilen Material, vorzugsweise aus Metall oder aus einer Metall-Legierung bestehende Elektrodenkörper 2 ist mechanisch stabil, verwindungsfrei und biegesteif sowie elektrisch isoliert innerhalb der Hohlkanüle 1 sowie des daran anschließenden Keramik-Hülsenkörpers 16 gefasst. Der distalseitig aus dem Keramik-Hülsenkörper 16 ragende Bereich B des Elektrodenkörpers 2 ist Löffel- bzw. Schaufelartig ausgebildet. An seinem distalen Ende 17 weist der Elektrodenkörper 2 eine wulstförmig ausgebildete und abgerundete Verdickung 22 auf. Die Elektrodenoberfläche 15 mündet proximalseitig bündig an der Aussenkontur des Keramik-Hülsenkörpers 16. Über eine Strecke I verläuft die Elektrodenoberfläche 15 im Wesentlichen parallel, geradlinig zur Längserstreckung des Keramik-Hülsenkörpers 16. Im Anschluss daran ist die Elektrodenoberfläche 15 konvex gekrümmt und mündet am distalen Ende 17 in der wulstförmig ausgebildeten und abgerundeten Verdickung 22.

Die Elektrodenoberfläche 14 ist im Wesentlichen löffelartig konkav geformt und weist an ihren beiden Längsseiten wulstartige Randkonturen 23 auf, deren Raumform in der Querschnittsdarstellung gemäß Fig. 10b längs der Schnittebene B-B ersichtlich ist.

Durch die beidseitigen Überhöhungen bedingt durch die wulstförmigen Randkonturen 23 gegenüber der mittig zur Sondenlängsachse angeordneten konkaven Vertiefung an der Elektrodenoberfläche 14 verleiht diese Formgebung dem Elektrodenkörper 2 eine erhöhte Form- bzw. Biegesteifigkeit insbesondere bei Krafteinwirkungen quer zur Sondenlängserstreckung. Die Oberflächenkontur der Elektrodenoberfläche 14 ähnelt der Außenkontur einer Acht, wohingegen die gegenüberliegende Elektrodenoberfläche 15 glatt ausgebildet ist.

Im distalen Bereich der wulstförmig ausgebildeten und abgerundeten Verdickung 22 längs der in Figur 10a ersichtlichen Schnittlinie C-C weist der Elektrodenkörper 2 die in Figur 10 c dargestellte ovale oder elliptische Querschnittsform auf.

Die radiale Erstreckung bzw. räumliche Ausdehnung des Elektrodenkörpers 5 überragt nicht die radiale Dimension der Hohlkanüle 1, die durch den Außendurchmesser b vorgegeben ist, so dass sichergestellt ist, dass die gesamte Sonde durch einen an die Hohlkanüle angepasst dimensionierten Arbeitskanal bspw. eines Resektoskops ungehindert geführt werden kann.

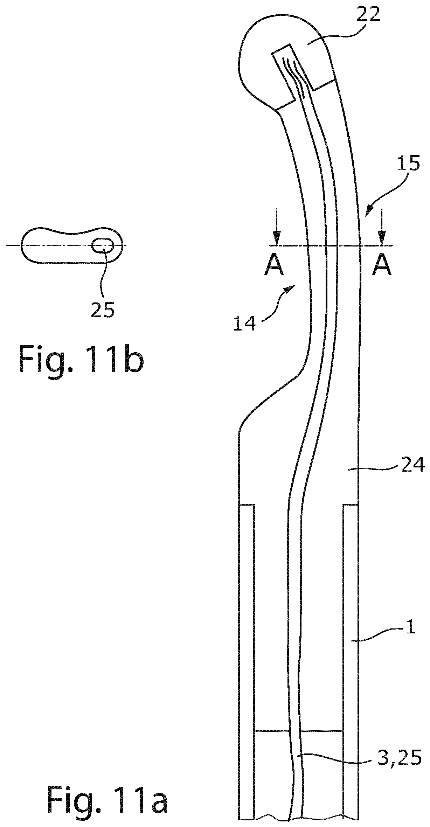

In Figur 11a ist ein Längsschnitt durch eine Sonde dargestellt, die anstelle des Keramik-Hülsenkörpers 16 sowie des Elektrodenkörpers 2, wie vorstehend zu Figur 10 erläutert, einen in die Hohlkanüle 1 hineinragenden und mit der Hohlkanüle 1 fest verbundenen Körper 24 aufweist, der mit Ausnahme der aus einem elektrisch leitenden Material gefertigten, wulstförmig ausgebildeten und abgerundeten Verdickung 22 aus einem elektrischen Isolator, vorzugsweise einer Keramik, oder einem Faserverstärkten Polymer, bspw. GFK, gefertigt ist und ansonsten die Form des in den vorstehenden Figuren 10 a, b dargestellten und erläuterten Elektrodenkörpers 2 besitzt. Durch den Körper 24 führt eine elektrische Leiteranordnung 3, die mit der elektrisch leitenden Verdickung 22 verbunden ist.

Die Querschnittsdarstellung gemäß Figur 11 b entspricht dem Querschnitt durch den Körper 24 längs der Schnittebene A-A gemäß Figur 11a und ist dem Querschnitt des Elektrodenkörpers 2 längs der Schnittebene B-B gemäß Figur 10b nachgebildet. Zusätzlich ist ein Durchführungskanal 25 für die elektrische Leiteranordnung 3 im Querschnitt vorgesehen.

Die in Figur 11a illustrierte Sonde ermöglicht eine nur auf den Bereich der wulstförmig ausgebildeten und abgerundeten Verdickung 22 begrenzte, bedarfsweise Applizierung von elektrischer Energie und eine damit erzielbare Koagulationswirkung auf das umliegende Gewebe. Alle übrigen Oberflächenbereiche des Körpers 24 sind elektrisch inaktiv.

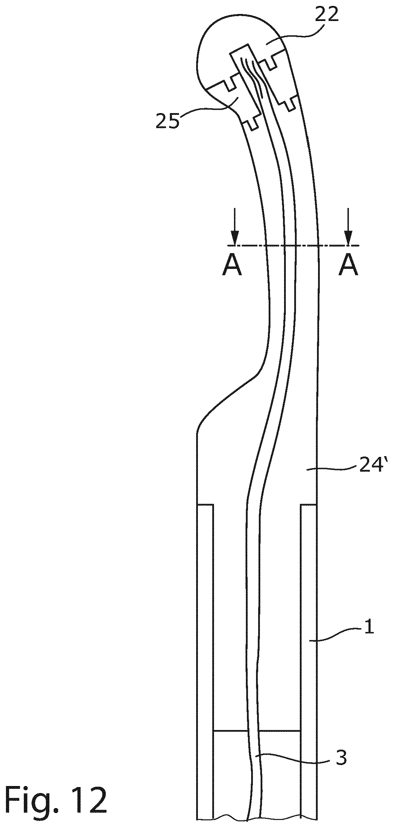

Alternativ zur Ausbildung des elektrisch isolierenden Körpers 24 ist in Figur 12 ein Längsschnitt durch ein Ausführungsbeispiel dargestellt, bei dem der Körper 24' aus einem metallischen, elektrisch leitfähigen Werkstoff gefertigt ist. Der Körper 24' ist an die Hohlkanüle 1 fest gefügt oder einstückig mit dieser verbunden. Distalseitig an den metallischen Körper 24' ist ein elektrischer Isolator 25 angebracht, an den elektrisch zum metallischen Körper 24' isoliert die aus einem metallischen Material bestehende, wulstförmig ausgebildete und abgerundete Verdickung 22 angebracht ist, die über die elektrische Leiteranordnung 3 mit elektrischer Energie bedarfsweise versorgbar ist. Der metallische Körper 24', dessen Raumform im Wesentlichen jener des vorstehend erläuterten Körpers 24 gemäß Figuren 11 a, b bzw. Elektrodenkörpers 2 gemäß Figur 10a entspricht, verfügt über ein hohes Maß an Robustheit und Biegesteifigkeit, insbesondere im Falle einer einstückigen bzw, monolithischen Ausbildung mit der Hohlkanüle 1. Der metallische Körper 24' weist längs der Schnittlinie A-A einen Querschnitt auf, der dem Querschnitt gemäß Figur 11b gleicht.

### Bezugszeichenliste

- 1: Sonde, Hohlkanüle
- 2: Elektrodenkörper
- 3: elektrischer Leiter
- 4: Führungshülse
- 5: erster axialer Abschnitt
- 6: zweiter axialer Abschnitt
- 7: Fügemittel
- 8: Kontakthülse
- 9: distales Ende
- 10: mamillenartige Kuppenform
- 11: gerader Abschnitt
- 12: gekrümmter Abschnitt
- 13: elektrisch islolierende Zwischenschicht
- 14,15: Elektrodenkörperoberfläche
- 16: Keramik-Hülsenkörper
- 17: distales Ende
- 18: Sondenlängsachse
- 19: Krümmungswendepunkt
- 20, 21: Oberflächenform
- 22: Verdickung
- 23: wulstartige Randkontur
- 24: elektrisch isolierende Körper
- 25: Durchführungskanal

## Patentansprüche

1. Vorrichtung zur Enukleation intrakorporaler Gewebebereiche, insbesondere der Prostata, mit einer Sonde (1), die als starre Hohlkanüle ausgebildet ist und an deren distalem Ende (9) wenigstens ein frei zugänglicher Elektrodenkörper (2) angebracht ist, der über wenigstens eine in Längserstreckung der Sonde verlaufende elektrische Leitung (3) mit elektrischer Energie beaufschlagbar ist, wobei
der Elektrodenkörper eine kuppenförmig ausgebildete Elektrodenoberfläche aufweist und orthogonal zur Sonden-Längserstreckung (z) orientierte Querschnittsflächen (A1-A6) besitzt, deren Flächengrößen längs eines ersten axialen Abschnittes (5), der das distale Kuppenende des Elektrodenkörpers enthält, mit zunehmenden Abstand zum distalen Kuppenende stetig zunehmen, und
wobei die Querschnittsflächen jeweils von einem Umfangsrand begrenzt sind, der an jeder Stelle stetig differenzierbar ist,
wobei die Sonde zumindest im Bereich des distalseitig mit der Sonde verbundenen Elektrodenkörpers über eine Biegefestigkeit verfügt, die unter Einwirken eines auf den Elektrodenkörper quer zur Längserstreckung der Sonde wirkenden Biegemomentes von wenigstens 0,1 Nm eine Formstabiliät der Sonde gewährleistet, und
wobei der frei zugängliche Elektrodenkörper Spatel-förmig ausgebildet ist und längs einer Querschnittsachse eines dem Elektrodenkörper zugeordneten Querschnittes derart abgeflacht ausgebildet ist, dass der Elektrodenkörper zwei abgeflachte Elektrodenkörperflächen (14, 15) aufweist.

2. Vorrichtung zur Enukleation intrakorporaler Gewebebereiche, insbesondere der Prostata, mit einer Sonde (1), die als starre Hohlkanüle ausgebildet ist und an deren distalem Ende (9) wenigstens ein frei zugänglicher Elektrodenkörper (2) angebracht ist, der über wenigstens eine in Längserstreckung der Sonde verlaufende elektrische Leitung (3) mit elektrischer Energie beaufschlagbar ist, wobei der Elektrodenkörper eine kuppenförmig ausgebildete Elektrodenoberfläche aufweist und orthogonal zur Sonden-Längserstreckung orientierte Querschnittsflächen besitzt, deren Flächengrößen längs eines ersten axialen Abschnittes, der das distale Kuppenende des Elektrodenkörpers enthält, mit zunehmenden Abstand zum distalen Kuppenende stetig zunehmen, und wobei die Querschnittsflächen jeweils von einem Umfangsrand begrenzt sind, der an jeder Stelle stetig differenzierbar ist, wobei die Sonde zumindest im Bereich des distalseitig mit der Sonde verbundenen Elektrodenkörpers über eine Biegefestigkeit verfügt, die unter Einwirken eines auf den Elektrodenkörper quer zur Längserstreckung der Sonde wirkenden Biegemomentes von wenigstens 0,1 Nm eine Formstabiliät der Sonde gewährleistet, wobei der frei zugängliche Elektrodenkörper Schaufel-förmig ausgebildet ist und längs einer Querschnittsachse eines dem Elektrodenkörper zugeordneten Querschnittes derart abgeflacht ausgebildet ist, dass der Elektrodenkörper zwei abgeflachte Elektrodenkörperflächen (14, 15) aufweist, von denen eine proximalseitig einen geraden Flächenabschnitt aufweist, an den distalseitig ein konvex gekrümmter Flächenabschnitt anschließt, und die andere konvex gekrümmt ausgebildet ist und distalseitig eine wulstförmig ausgebildete und abgerundete Verdickung (22, 23) aufweist.

3. Vorrichtung nach Anspruch 1 oder 2, wobei zumindest der Bereich des distalseitig mit der Sonde verbundenen Elektrodenkörpers sich von dessen distalen Elektrodenspitze bis maximal 30 mm proximalwärts längs der Sonde erstreckt.

4. Vorrichtung nach einem der Ansprüche 1 bis 3, wobei der Elektrodenkörper aus Metall oder einer Metalllegierung gefertigt ist, der in Form einer Monoplar-Elektrode mit der längs der Sonde geführten, elektrischen Leitung oder in Form einer Bipolar-Elektrode mit zwei längs der Sonde geführten, elektrischen Leitungen elektrisch verbunden ist.

5. Vorrichtung nach einem der Ansprüche 1 bis 4, wobei längs der Sonde eine Führungshülse angebracht ist, zum Durchführen eines medizinischen Instrumentes parallel zur Sonde und/oder als Zentrier- und Gleitelement innerhalb und längs eines Arbeitskanals eines Resektoskops.

6. Vorrichtung nach einem der Ansprüche 1 bis 5,
wobei die Elektrodenoberfläche des Elektrodenkörpers, und/oder die Sonde mit einer reibungsarmen Beschichtung überzogen ist und
wobei die Beschichtung PTFE, TPU, Polysiloxane oder Hydrogel aufweist.

7. Vorrichtung nach einem der Ansprüche 1, 3 bis 6, wobei die zwei abgeflachten Elektrodenkörperoberflächen eine der nachfolgenden Form-Paare besitzen:
beide Elektrodenkörperoberflächen weisen wenigstens einen ebenen Flächenbereiche auf,
beide Elektrodenkörperoberflächen weisen wenigstens einen konvex gekrümmten Flächenbereich auf,
eine Elektrodenkörperoberfläche weist wenigstens einen konvex gekrümmten Flächenbereich und die andere Elektrodenkörperoberfläche wenigstens einen konkav gekrümmten Flächenbereich auf,
eine der beiden Elektrodenkörperoberflächen weist wenigstens einen gekrümmten Flächenbereich und die andere Elektrodenkörperoberfläche wenigstens einen ebenen Flächenbereich auf.

8. Vorrichtung nach einem der Ansprüche 1, 3 bis 7, wobei der frei zugängliche Elektrodenkörper einen ovalförmigen Querschnitt aufweist, der zu einer dem Oval zuordenbaren Längsachse symmetrisch ausgebildet ist.

9. Vorrichtung nach Anspruch 8, wobei der ovalförmige Querschnitt zu einer die Längsachse orthogonalen Achse unsymmetrisch ausgebildet ist.

10. Vorrichtung nach Anspruch 2, wobei der frei zugängliche Elektrodenkörper in axialer Projektion zur starren Hohlkanüle die Hohlkanüle radial nicht überragt.

11. Vorrichtung nach Anspruch 2, wobei der frei zugängliche Elektrodenkörper einen Querschnitt aufweist, der zumindest bereichsweise die Außenform einer Acht aufweist.

12. Vorrichtung zur Enukleation intrakorporaler Gewebebereiche, insbesondere der Prostata, mit einer Sonde (1), die als starre Hohlkanüle ausgebildet ist und an deren distalem Ende (9) wenigstens ein frei zugänglicher Elektrodenkörper (2, 22) angebracht ist, der über wenigstens eine in Längserstreckung der Sonde verlaufende elektrische Leitung (3) mit elektrischer Energie beaufschlagbar ist, wobei der Elektrodenkörper eine kuppenförmig ausgebildete Elektrodenoberfläche aufweist und orthogonal zur Sonden-Längserstreckung (z) orientierte Querschnittsflächen besitzt, deren Flächengrößen längs eines ersten axialen Abschnittes, der das distale Kuppenende des Elektrodenkörpers enthält, mit zunehmenden Abstand zum distalen Kuppenende stetig zunehmen, wobei die Querschnittsflächen jeweils von einem Umfangsrand begrenzt sind, der an jeder Stelle stetig differenzierbar ist, wobei die Sonde zumindest im Bereich des distalseitig mit der Sonde verbundenen Elektrodenkörpers über eine Biegefestigkeit verfügt, die unter Einwirken eines auf den Elektrodenkörper quer zur Längserstreckung der Sonde wirkenden Biegemomentes von wenigstens 0,1 Nm eine Formstabiliät der Sonde gewährleistet, und wobei distalseitig an der starren Hohlkanüle ein spatel- oder schaufelförmig ausgebildeter Formkörper (24) angebracht ist, an dem distalseitig der frei zugängliche Elektrodenkörper (22) angebracht ist.

13. Vorrichtung nach Anspruch 12,
wobei der Formkörper aus einem elektrisch isolierenden Material besteht, an dem distalseitig der frei zugängliche Elektrodenkörper unmittelbar angebracht ist, oder
wobei der Formkörper aus einem elektrisch leitenden Material besteht, an dem distalseitig der frei zugängliche Elektrodenkörper über einen elektrischen Isolator angebracht ist.

14. Vorrichtung nach Anspruch 12 oder 13,
wobei der Formkörper Schaufel-förmig ausgebildet ist, mit einer abgeflachten Elektrodenkörperfläche, die proximalseitig einen geraden Flächenabschnitt aufweist, an den distalseitig ein konvex gekrümmter Flächenabschnitt anschließt,
dass die andere abgeflachte Elektrodenkörperoberfläche konvex gekrümmt ausgebildet ist, und
wobei mittel- oder unmittelbar distalseitig am Formkörpern der frei zugängliche Elektrodenkörper in Form einer wulstförmig ausgebildeten und abgerundeten Verdickung anschließt.

15. Vorrichtung nach einem der Ansprüche 12 bis 14, wobei der Formkörper einen Querschnitt aufweist, der zumindest bereichsweise die Außenform einer Acht aufweist.

## Claims

1. Device for enucleation of intracorporeal tissue areas, in particular the prostate, with a probe (1) which is designed as a rigid hollow cannula and at whose distal end (9) at least one freely-accessible electrode body (2) is attached, which can be supplied with electrical energy via at least one electrical line extending in the longitudinal direction of the probe (3), wherein
the electrode body has a dome-shaped electrode surface and cross-sectional areas (A1-A6) oriented orthogonally to the longitudinal axis (z) of the probe, the surface areas of which increase steadily along a first axial section (5) which contains the distal dome end of the electrode body, with increasing distance from the distal dome end, and
wherein
the cross-sectional areas are each bounded by a peripheral edge which is continuously differentiable at every point,
wherein
the probe has a bending strength, at least in the region of the electrode body connected to the probe on the distal side, which ensures the dimensional stability of the probe under the action of a bending moment of at least 0.1 Nm acting on the electrode body transversely to the longitudinal extension of the probe, and
wherein
the freely-accessible electrode body is spatula-shaped and is flattened along a cross-sectional axis of a cross-section associated with the electrode body in such a way that the electrode body has two flattened electrode body surfaces (14, 15).

2. Device for enucleation of intracorporeal tissue areas, in particular the prostate, with a probe (1), which is designed as a rigid hollow cannula and to whose distal end (9) at least one freely-accessible electrode body (2) is attached, which can be supplied with electrical energy via at least one electrical conductor (3) extending in the longitudinal direction of the probe, wherein the electrode body has a dome-shaped electrode surface and cross-sectional surfaces oriented orthogonally to the longitudinal extension of the longitudinal extension of the probe, the surface areas of which increase continuously along a first axial section, which contains the distal dome end of the electrode body, with increasing distance from the distal dome end, and wherein the cross-sectional areas are each bounded by a peripheral edge which is continuously differentiable at every point,
wherein
the probe has a bending strength, at least in the region of the electrode body connected to the probe on the distal side, which ensures the dimensional stability of the probe under the action of a bending moment of at least 0.1 Nm acting on the electrode body transversely to the longitudinal extension of the probe,
wherein
the freely-accessible electrode body is designed in a shovel shape and is flattened along a cross-sectional axis of a cross-section assigned to the electrode body in such a way that the electrode body has two flattened electrode body surfaces, one of which has a straight surface section on the proximal side, which is connected to a convexly-curved surface section on the distal side, and the other is convexly-curved and has a bead-shaped and rounded thickening (22, 23) on the distal side.

3. Device according to claim 1 or 2,
wherein
at least the region of the electrode body connected to the probe on the distal side extends from its distal electrode tip to a maximum of 30 mm proximally along the probe.

4. Device according to any one of claims 1 to 3,
wherein
the electrode body is made of metal or a metal alloy, which is electrically connected in the form of a monopolar electrode with the electrical conductor guided along the probe or in the form of a bipolar electrode with two electrical conductors guided along the probe.

5. Device according to any one of claims 1 to 4,
wherein
a guide sleeve is attached along the probe for guiding a medical instrument parallel to the probe and/or as a centering and sliding element within and along a working channel of a resectoscope.

6. Device according to any one of claims 1 to 5,
wherein
the electrode surface of the electrode body and/or the probe is coated with a low-friction coating and
wherein
the coating comprises TFE, TPU, polysiloxanes or hydrogel.

7. Device according to any one of claims 1, 3 to 6,
wherein
the two flattened electrode body surfaces have one of the following pairs of shapes:
both electrode body surfaces have at least one flat surface area,
both electrode body surfaces have at least one convexly-curved surface area,
one electrode body surface has at least one convexly-curved surface area and the other electrode body surface has at least one concavely curved surface area,
one of the two electrode body surfaces has at least one curved surface area and the other electrode body surface has at least one flat surface area.

8. Device according to any one of claims 1, 3 to 7,
wherein
the freely-accessible electrode body has an oval-shaped cross-section that is symmetrical with respect to a longitudinal axis associated with the oval.

9. Device according to claim 8,
wherein
the oval-shaped cross-section is asymmetrical with respect to an axis orthogonal to the longitudinal axis.

10. Device according to claim 2,
wherein
the freely-accessible electrode body does not protrude radially beyond the rigid hollow cannula in the axial projection.

11. Device according to claim 2,
wherein
the freely-accessible electrode body has a cross-section which, at least in some areas, has the outer shape of a figure eight.

12. Device for enucleation of intracorporeal tissue areas, in particular the prostate, with a probe (1) which is designed as a rigid hollow cannula and at whose distal end (9) at least one freely-accessible electrode body (2, 22) is attached, which can be supplied with electrical energy via at least one electrical conductor (3) extending in the longitudinal direction of the probe, wherein the electrode body has a dome-shaped electrode surface and cross-sectional areas oriented orthogonally to the longitudinal direction of the probe (z) of the probe, the surface areas of which increase continuously along a first axial section containing the distal dome end of the electrode body with increasing distance from the distal dome end, wherein the cross-sectional areas are each bounded by a peripheral edge that is continuously differentiable at every point,
wherein the probe has a bending strength, at least in the region of the electrode body connected to the probe on the distal side, which ensures the dimensional stability of the probe under the action of a bending moment of at least 0.1 Nm acting on the electrode body transversely to the longitudinal extension of the probe, and
wherein
a spatula- or shovel-shaped molded body is attached distally to the rigid hollow cannula, to which the freely-accessible electrode body is attached distally.

13. Device according to claim 12, wherein the molded body consists of an electrically insulating material, to which the freely-accessible electrode body is directly attached on the distal side, or wherein the molded body consists of an electrically conductive material, to which the freely-accessible electrode body is attached on the distal side via an electrical insulator.

14. Device according to claim 12 or 13,
wherein
the molded body is shaped like a shovel, with a flattened electrode body surface that has a straight surface section on the proximal side, which is connected to a convexly-curved surface section on the distal side,
the other flattened electrode body surface is convexly-curved, and wherein the freely-accessible electrode body adjoins the molded body on the medial or immediate distal side in the form of a bead-shaped and rounded thickening.

15. Device according to any one of claims 12 to 14, wherein the molded body has a cross-section which, at least in some areas, has the outer shape of a figure eight.

## Revendications

1. Dispositif d'énucléation de zones tissulaires intracorporelles, en particulier de la prostate, comprenant une sonde (1), qui est constituée sous la forme d'une canule creuse, rigide et à l'extrémité (9) distale de laquelle est monté au moins un corps (2) d'électrode accessible librement, qui peut être alimenté en énergie électrique par au moins une ligne (3) électrique s'étendant suivant l'étendue longitudinale de la sonde, dans lequel le corps d'électrode a une surface d'électrode constituée en forme de calotte et possède des surfaces (A1 - A6) de section transversale orientées orthogonalement à l'étendue (z) longitudinale de la sonde, dont la dimension de surface le long d'une premier partie (5) axiale, qui contient l'extrémité distale de la calotte du corps d'électrode, augmente constamment au fur et à mesure qu'augmente la distance à l'extrémité distale de la calotte, et
dans lequel les surfaces de section transversale sont délimitées respectivement par un bord de pourtour, qui peut être différencié constamment en chaque point,
dans lequel la sonde dispose, au moins dans la zone du corps d'électrode relié du côté distal à la sonde, d'une résistance à la flexion, qui assure une stabilité de forme de la sonde d'au moins 0,1 Nm sous l'effet d'un couple de flexion s'appliquant au corps d'électrode transversalement à l'étendue longitudinale de la sonde, et
dans lequel le corps d'électrode accessible librement est constitué en forme de spatule et est le long d'un axe d'une section transversale, associée au corps d'électrode, de constitution aplatie de manière à ce que le corps d'électrode ait deux surfaces (14, 15) de corps d'électrode aplaties.

2. Dispositif d'énucléation de zones tissulaires intracorporelles, en particulier de la prostate, comprenant une sonde (1), qui est constituée sous la forme d'une canule creuse, rigide et à l'extrémité (9) distale de laquelle est monté au moins un corps (2) d'électrode accessible librement, qui peut être alimenté en énergie électrique, par au moins une ligne (3) électrique s'étendant suivant l'étendue longitudinale de la sonde, dans lequel le corps d'électrode a une surface d'électrode constituée en forme de calotte et possède des surfaces de section transversale orientées orthogonalement à l'étendue longitudinale de la sonde dont la dimension de surface le long d'une premier partie axiale, qui contient l'extrémité distale de la calotte du corps d'électrode, augmente constamment au fur et à mesure qu'augmente la distance à l'extrémité distale de la calotte, et dans lequel les surfaces de section transversale sont délimitées respectivement par un bord de pourtour, qui peut être différencié constamment en chaque point,
dans lequel la sonde dispose au moins dans la zone du corps d'électrode, relié du côté distal à la sonde, d'une résistance à la flexion, qui assure une stabilité de forme de la sonde d'au moins 0,1 Nm sous l'effet d'un couple de flexion s'appliquant au corps d'électrode transversalement à l'étendue longitudinale de la sonde, et
dans lequel le corps d'électrode accessible librement est constitué en forme de spatule et est le long d'un axe d'une section transversale, associée au corps d'électrode, de constitution aplatie de manière à ce que le corps d'électrode ait deux surfaces (14, 15) de corps d'électrode aplaties,
dont l'une a, du côté proximal, une partie de surface droite, à laquelle se raccorde du côté distal une partie de surface convexe, et l'autre est de constitution convexe et a du côté
distal un épaississement (22, 23) constitué en forme de bourrelet et arrondi.

3. Dispositif suivant la revendication 1 ou 2,
dans lequel au moins la zone du corps d'électrode relié du côté distal à la sonde s'étend de sa pointe d'électrode distale jusqu'à 30 mm au maximum du côté proximal le long de la sonde.

4. Dispositif suivant l'une des revendications 1 à 3,
dans lequel le corps d'électrode est en métal ou en un alliage métallique qui, sous la forme d'une électrode monopolaire, est relié électriquement à la ligne électrique passant le long de la sonde ou sous la forme d'une électrode bipolaire est relié électriquement à deux lignes électriques passant le long de la sonde.

5. Dispositif suivant l'une des revendications 1 à 4,
dans lequel le long de la sonde est monté un manchon de guidage pour le passage d'un instrument médical parallèlement à la sonde et/ou comme élément de centrage et de glissement à l'intérieur et le long d'un canal de travail d'un résectoscope.

6. Dispositif suivant l'une des revendications 1 à 5,
dans lequel la surface d'électrode du corps d'électrode et/ou la sonde est revêtue d'un revêtement à petit frottement et dans lequel le revêtement a du PTFE, du TPU, du polysiloxane ou un hydrogel.

7. Dispositif suivant l'une des revendications 1, 3 à 6,
dans lequel les deux surfaces aplaties du corps d'électrode possèdent l'une des paires de forme suivantes :
les deux surfaces du corps d'électrode ont au moins une zone de surface plane,
les deux surfaces du corps d'électrode ont au moins une zone de surface convexe,
une surface du corps a au moins une zone de surface convexe et l'autre surface du corps d'électrode a au moins une zone de surface concave,
l'une des deux surfaces du corps d'électrode a au moins une zone de surface incurvée et l'autre surface du corps d'électrode a au moins une zone de surface plane.

8. Dispositif suivant l'une des revendications 1, 3 à 7,
dans lequel le corps d'électrode accessible librement a une section transversale de forme ovale, qui est constituée symétriquement par rapport à un axe longitudinal pouvant être associé à l'ovale.

9. Dispositif suivant la revendication 8,
dans lequel la section transversale de forme ovale est constituée de manière dissymétrique par rapport à un axe orthogonal à l'axe longitudinal.

10. Dispositif suivant la revendication 2,
dans lequel le corps d'électrode accessible librement ne dépasse pas, en projection axiale par rapport à la canule creuse, rigide, radialement de la canule creuse.

11. Dispositif suivant la revendication 2,
dans lequel le corps d'électrode accessible librement a une section transversale qui, au moins par endroit, a la forme extérieure d'un huit.

12. Dispositif d'énucléation de zones tissulaires intracorporelles, en particulier de la prostate, comprenant une sonde (1), qui est constituée sous la forme d'une canule creuse, rigide et à l'extrémité (9) distale de laquelle est monté au moins un corps (2, 22) d'électrode accessible librement, qui peut être alimenté en énergie électrique par au moins une ligne (3) électrique s'étendant suivant l'étendue longitudinale de la sonde, dans lequel le corps d'électrode a une surface d'électrode constituée en forme de calotte et possède des surfaces de section transversale orientées orthogonalement à l'étendue (z) longitudinale de la sonde, dont la dimension de surface le long d'une premier partie axiale, qui contient l'extrémité distale de la calotte du corps d'électrode, augmente constamment au fur et à mesure qu'augmente la distance à l'extrémité de la calotte distale, et
dans lequel les surfaces de section transversale sont délimitées respectivement par un bord de pourtour, qui peut être différencié constamment en chaque point,
dans lequel la sonde dispose, au moins dans la zone du corps d'électrode relié du côté distal à la sonde, d'une résistance à la flexion, qui assure une stabilité de forme de la sonde d'au moins 0,1 Nm sous l'effet d'un couple de flexion s'appliquant au corps d'électrode transversalement à l'étendue longitudinale de la sonde, et
dans lequel du côté distal, sur la canule creuse, rigide, est monté un corps (24) de forme, constitué en forme de spatule ou de pale et sur lequel est monté du côté distal le corps (22) d'électrode accessible librement.

13. Dispositif suivant la revendication 12,
dans lequel le corps de forme est en un matériau isolant électriquement sur lequel, du côté distal, le corps d'électrode accessible librement est monté directement, ou
dans lequel le corps de forme est en un matériau conducteur de l'électricité sur lequel, du côté distal, le corps d'électrode accessible librement est monté par l'intermédiaire d'un isolant électrique.

14. Dispositif suivant la revendication 12 ou 13,
dans lequel le corps de forme est constitué en forme de pale, comprenant une première surface aplatie de corps d'électrode qui a, du côté proximal, une partie de surface droite, à laquelle se raccorde, du côté distal, une partie de surface convexe,
en ce que l'autre surface aplatie du corps d'électrode est convexe, et
dans lequel directement ou indirectement, du côté distal, sur le corps de forme, le corps d'électrode accessible librement se raccorde sous la forme d'un épaississement constitué en forme de bourrelet et arrondi.

15. Dispositif suivant l'une des revendications 12 à 14,
dans lequel le corps de forme a une section transversale qui a, au moins, par endroit, la forme extérieure d'un huit.
